# EUROPÄISCHE PATENTANMELDUNG

(11) **EP 1 192 900 A2**
(43) Veröffentlichungstag der Anmeldung: **03.04.2002**
(21) Anmeldenummer: 01122931.7
(22) Anmeldetag: 25.09.2001
(51) Int. Cl.: A61B 6/03

(54) **Vorrichtung und Verfahren zur Erzeugung eines Röntgen-Computertomogramms mit einer Streustrahlungskorrektur**

(30) Priorität: 27.09.2000 DE 10047720
(71) Anmelder: Philips Corporate Intellectual Property GmbH, 52064 Aachen (DE); Koninklijke Philips Electronics N.V., 5621 BA Eindhoven (NL)
(72) Erfinder: Schneider, Stefan, Dr., Habsburgerallee 11, 52064 Aachen (DE); Lauter, Josef, Dr., Habsburgerallee 11, 52064 Aachen (DE); Wieczorek, Herfried, Dr., Habsburgerallee 11, 52064 Aachen (DE); Such, Olaf, Dr., Habsburgerallee 11, 52064 Aachen (DE)
(74) Vertreter: Volmer, Georg, Dipl.-Ing.

(57) **Zusammenfassung**

Die Erfindung betrifft Verfahren zur Streustrahlungskorrektur bei der Erzeugung eines Röntgen-Computertomogramms. Dabei wird die Verteilung der Streustrahlung mit Hilfe der Detektorzellen (7') bestimmt, die in einem zweidimensionalen mehrzelligen Detektorfeld (3) aufgrund des durchgeführten Messverfahrens von der direkten Bestrahlung abgeschirmt sind. Mit Hilfe dieser Verteilung wird dann einen Streustrahlungskorrektur in den benachbarten, direkt bestrahlten Detektorzellen (7) vorgenommen. Ferner kann eine Streustrahlungskorrektur mit Hilfe einer Computersimulation der Streuprozesse erfolgen. Dabei wird vorzugsweise ein Monte-Carlo-Verfahren angewendet, und es wird der Einfluss der Geometrie und des Materialaufbaus der Messanordnung, der Patientengröße, des durchstrahlten Gewebebereichs und dergleichen berücksichtigt.

## Beschreibung

Die Erfindung betrifft ein Verfahren zur Erzeugung eines Röntgen-Computertomogramms eines Objektes, wobei das Objekt in einer Messanordnung bestehend aus einer Röntgenstrahlungsquelle und einem Detektorfeld durchstrahlt wird und die im Detektorfeld gemessene Strahlungsintensität einer Streustrahlungskorrektur unterzogen wird. Dabei kann insbesondere das Detektorfeld zweidimensional mehrzellig und ein nicht rechtwinklig begrenzter Teil des Detektorfeldes von der direkten Bestrahlung durch die Röntgenstrahlungsquelle abgeschirmt sein. Die Erfindung betrifft ferner einen Röntgen-Computertomographen mit einer Messanordnung, die eine Röntgenstrahlungsquelle und ein Detektorfeld enthält, sowie mit einer Korrektureinheit zur Durchführung einer Streustrahlungskorrektur an der im Detektorfeld gemessenen Strahlungsintensität. Insbesondere betrifft sie einen solchen Röntgen-Computertomographen, bei dem das Detektorfeld zweidimensional mehrzellig ist und Abschirmungsmittel enthält, die so angeordnet sind, dass sie einen nicht rechtwinklig begrenzten Teil des Detektorfeldes abschirmen.

Bei der Erzeugung eines Computertomogramms wird ein zu untersuchendes Objekt wie insbesondere der Körper eines Patienten von einer Röntgenstrahlungsquelle mit Röntgenstrahlung durchstrahlt, und die durchtretende Strahlung wird auf der anderen Seite des Objektes in einem Detektorfeld bezüglich ihrer Strahlungsintensität erfasst. Zur Erzeugung eines Querschnittbildes des durchstrahlten Objektes wird aus der im Detektorfeld gemessenen Strahlungsintensität auf die Absorption des Röntgenstrahls auf seinem Weg durch das Objekt und damit auf die optische Dichte (gemessen in Hounsfield-Einheiten) beziehungsweise Materialzusammensetzung des Objektes geschlossen.

Für die Erzeugung der gewünschten Abbildungsinformation ist die das Objekt direkt durchtretende (Primär-)Strahlung verantwortlich. Daneben finden im Objekt jedoch auch noch Streuprozesse der Photonen der Röntgenstrahlung statt, bei welchen die Photonen ihre Richtung und gegebenenfalls ihre Energie ändern. Die gestreute Strahlung erreicht ebenfalls zum Teil den Detektor und trägt dort zur gemessenen Strahlungsintensität bei. Da die Streustrahlung jedoch nicht auf direktem Wege von der Strahlungsquelle zum Detektor gelangt ist, trägt sie nicht zur verwertbaren Bildinformation bei, sondern überlagert vielmehr die aus der direkten Strahlung erhältliche Information.

Da die Streustrahlung in erster Näherung linear mit dem durchstrahlten Volumen des Objektes zunimmt, steigt ihr störender Einfluss mit zunehmender Scheibendicke, die in dem Computertomographen (CT) durchstrahlt wird. Während derzeit in Computertomographen typischerweise Scheibendicken von 0.8 - 3 mm untersucht werden, besteht ein zunehmender Trend zum Einsatz von mehrzeiligen Computertomographen, die eine Scheibendicke von etwa 2 cm oder sogar noch mehr haben. Dementsprechend wird der Hintergrund durch Streustrahlung und die dadurch verursachte Verschlechterung der Bildqualität zunehmen.

Zur Reduzierung des störenden Einflusses der Streustrahlung sind verschiedene Verfahren bekannt. So kann zum einen zu verhindern versucht werden, dass die Streustrahlung den Detektor überhaupt erreicht. Diese Verfahren nutzen den einzigen relevanten Unterschied zwischen primären Photonen und gestreuten Photonen aus, der in der Verteilung ihrer Einfallswinkel besteht. Während alle primären Photonen auf geradem Wege von der Strahlungsquelle kommen, weisen gestreute Photonen hiervon abweichende "schräge" Einfallswinkel auf. Es ist daher bekannt, sogenannte Anti-Streu-Gitter (ASG) einzusetzen, die aus dünnen Folien eines hochgradig absorbierenden Materials wie zum Beispiel Molybdän oder Wolfram bestehen und ausgerichtet auf den Brennpunkt der Röntgenanode angeordnet werden. Das ASG unterdrückt daher primäre Photonen nur dann, wenn sie auf eine Stirnfläche der Folien treffen, während gestreute Photonen bei ihrem Auftreffen auf die Folienfläche absorbiert werden. Für computertomographische Anwendungen kann der Unterdrückungsfaktor der Streustrahlung hierdurch leicht hohe Werte von 10 bis 20 annehmen. Im Gegensatz dazu wird die primäre Strahlung nur um einen Faktor von etwa 1.1 bis 1.3 reduziert. Nachteilig an den Anti-Streu-Gittern ist jedoch, dass sie hohe Herstellungskosten verursachen und aufwendig im Einsatz sind. Ferner ist fraglich, ob die erzielten Unterdrückungsfaktoren für zukünftige hohe Schichtdicken tatsächlich ausreichend sind.

Weiterhin findet eine Streustrahlungskorrektur üblicherweise dadurch statt, dass ein konstanter Streustrahlungshintergrund, der aus der Objektgröße konservativ abgeschätzt wird, von den Messwerten abgezogen wird. Bildartefakte wie Streifen und die sogenannte Tassenbildung bleiben jedoch sichtbar.

Aus der US 5 615 279 ist ein Verfahren zur Streustrahlungskorrektur an einem Computertomographen bekannt, bei welchem zunächst Messungen der Streustrahlung an verschieden dicken Modellkörpern (Phantomen) durchgeführt und in einer Tabelle abgespeichert werden. Bei der Erzeugung von Computertomogrammen an realen Patienten werden dann unter Rückgriff auf die abgespeicherten Tabellen Korrekturwerte für die Messdaten berechnet, um den Einfluss der Streustrahlung möglichst weitgehend zu eliminieren. Nachteilig bei diesem Verfahren ist der hohe experimentelle Aufwand, der zur Erzeugung der Tabellen mit den Korrekturwerten erforderlich ist. Aufgrund dieses hohen Aufwandes können in der Regel nur wenige Parameter variiert werden, die Einfluss auf die Streustrahlung haben. Hierzu gehört insbesondere die Objektgröße des Phantoms.

Des weiteren ist aus der DE 197 21 535 A1 ein Röntgen-Computertomograph bekannt, bei welchem das Detektorfeld aus mehreren nebeneinander liegenden Zeilen bestehend aus aneinandergereihten Detektorzellen gebildet wird. Die aus der Röntgenstrahlungsquelle und dem Detektorfeld sowie Kollimatoren bestehende Messanordnung ist relativ zur Längsachse des Patienten verschiebbar. Die mehreren Zeilen des Detektorfeldes sind dabei quer zur Verschiebungsachse und parallel nebeneinander angeordnet, so dass bei einer Relativverschiebung zwischen Messanordnung und Patient nacheinander verschiedene Schichten des Körpers auf den Detektorzellen abgebildet werden. Die Kollimatoren können insbesondere so eingestellt werden, dass die am Rand des Detektorfeldes gelegenen Detektorzeilen von der direkten Bestrahlung durch die Röntgenstrahlungsquelle abgeschirmt werden. In diesen Zeilen kann somit nur Streustrahlung auftreffen, so dass das dort registrierte Messsignal ein Indiz für die Größe der Streustrahlung liefert und für eine Streustrahlungskorrektur der primären Messwerte verwendet werden kann. Nachteilig bei dieser Anordnung ist, dass für die Streustrahlungskorrektur eigene Zeilen des Detektorfeldes bereitgestellt werden müssen.

Vor diesem Hintergrund war es Aufgabe der vorliegenden Erfindung, ein Verfahren zur Erzeugung eines Röntgen-Computertomogramms sowie einen Röntgen-Computertomographen bereitzustellen, mit welchen eine verbesserte Streustrahlungskorrektur möglich ist.

Diese Aufgabe wird durch ein Verfahren mit den Merkmalen des Anspruchs 1, ein Verfahren mit den Merkmalen des Anspruchs 10, einen Röntgen-Computertomographen mit den Merkmalen des Anspruchs 7 sowie einen Röntgen-Computertomographen mit den Merkmalen des Anspruchs 18 gelöst. Vorteilhafte Ausgestaltungen sind in den Unteransprüchen enthalten.

Gemäß einer ersten Ausführungsform der Erfindung ist ein Verfahren zur Erzeugung eines Röntgen-Computertomogramms von einem Objekt wie insbesondere einem Patientenkörper vorgesehen, bei welchem das Objekt in einer Messanordnung bestehend aus einer Röntgenstrahlungsquelle und einem Detektorfeld durchstrahlt wird und die im Detektorfeld gemessene Strahlungsintensität einer Streustrahlungskorrektur unterzogen wird. Das Detektorfeld ist dabei zweidimensional mehrzellig ausgebildet, das heißt, dass die einzelnen Zellen des Detektors matrixförmig in Zeilen und Spalten nebeneinander angeordnet sind. Dabei müssen nicht alle Zellen geometrisch die gleiche Größe aufweisen.

Weiterhin wird bei dem Verfahren ein nicht rechtwinklig begrenzter Teil des Detektorfeldes von der direkten Bestrahlung durch die Röntgenstrahlungsquelle abgeschirmt. Derartige Abschirmungen treten bei verschiedenen Verfahren zur Erzeugung eines Computertomogramms auf, bei denen zum Beispiel aufgrund einer besonderen Relativbewegung zwischen Messanordnung und Objekt die Auswertung der Messsignale detektorseitig in einem nicht rechtwinklig begrenzten Bereich erfolgen muss. Ein Beispiel für ein solches Verfahren ist ein helixförmiges Scannen des Objektes, bei welchem die Messanordnung auf einer Spiralbahn entlang der Achse des Objektes fortschreitend um das Objekt rotiert. Der für die Auswertung der Röntgenaufnahme relevante Teil des Detektorfeldes nimmt bei solchen Verfahren kompliziertere Formen an, die nicht in einfacher Weise rechtwinklig begrenzt sind (vgl. US 5,463,666 und DE 198 44 543 A1).

Erfindungsgemäß wird bei einem derartigen Verfahren zur Erzeugung eines Röntgen-Computertomogramms auch die Strahlungsintensität im abgeschirmten Teil des Detektorfeldes gemessen und für die Streustrahlungskorrektur der Messwerte im direkt bestrahlten Bereich des Detektorfeldes verwendet. Anders als bei üblichen Verfahren wird somit das Signal des abgeschirmten Teils des Detektorfeldes nicht verworfen. Da auf den abgeschirmten Teil des Detektorfeldes keine direkte Primärstrahlung fällt, trägt dieser Teil nicht zur eigentlichen Bildinformation bei. Die in diesem Teil registrierte Strahlungsintensität rührt allein aus Streustrahlung her. Die Messwerte in diesem Teil können somit einen Anhaltspunkt für die lokale Größe der Streustrahlung liefern, so dass sie bei der Korrektur der eigentlichen Messwerte aus dem direkt bestrahlten Teil des Detektorfeldes vorteilhaft verwendet werden können. Für diese Streustrahlungskorrektur ist es nicht erforderlich, zusätzliche Zeilen im Detektorfeld vorzusehen. Vielmehr werden die aufgrund der gewählten Abschirmung ohnehin vorhandenen, nicht direkt bestrahlten Zellen des Detektorfeldes dazu ausgenutzt, die gewünschte Information über die Streustrahlung zu ermitteln.

Der abgeschirmte Teil des Detektorfeldes ist zumindest teilweise krummlinig begrenzt. Eine derartige krummlinige Begrenzung ergibt sich bei verschiedenen Verfahren zur Röntgen-Computertomographie aufgrund der Relativbewegung von Messanordnung und Objekt.

Gemäß einer Weiterbildung des Verfahrens werden bei der Streustrahlungskorrektur nur solche Zellen des abgeschirmten Teils des Detektorfeldes berücksichtigt, welche vollständig, d.h. über ihre ganze Fläche abgeschirmt sind. Die Zellen des Detektorfeldes sind in der Regel rechtwinklig beziehungsweise quadratisch und im Detektorfeld in einem regelmäßigen Raster angeordnet. Demgegenüber ist derjenige Teil des Detektorfeldes, welcher durch eine entsprechende Anordnung von Abschirmungsmitteln von der direkten Bestrahlung durch die Röntgenstrahlungsquelle abgeschirmt ist, durch nicht zueinander rechtwinklig stehende Seiten oder sogar durch krummlinig verlaufende Seiten begrenzt. Das führt dazu, dass die Ränder der abgeschirmten Fläche des Detektorfeldes in der Regel mitten durch die hiervon tangierten Zellen des Detektorfeldes verlaufen. Diese Zellen liegen daher teilweise im abgeschirmten und teilweise im direkt bestrahlten Bereich.

Darüber hinaus ist ein Halbschatten vorhanden, welcher durch die endliche Ausdehnung der Röntgenstrahlungsquelle verursacht wird. Die an der Grenze zwischen abgeschirmtem und nicht abgeschirmtem Teil des Detektorfeldes liegenden Zellen des Detektors liefern daher in der Regel ein Signal, welches sich nicht eindeutig dem einen oder anderen Teil zuordnen lässt. Um Messfehler zu vermeiden, werden die Signale dieser Detektorzellen, die nicht vollständig abgeschirmt sind, somit vorzugsweise bei der Streustrahlungskorrektur nicht berücksichtigt.

Gemäß einer anderen Weiterbildung der Erfindung wird die über zwei oder mehr Zellen des Detektorfeldes gemittelte Strahlungsintensität im abgeschirmten Teil des Detektorfeldes für die Streustrahlungskorrektur verwendet. Da sich die Größe der Streustrahlung in der Regel niederfrequent, das heißt räumlich nur langsam ändert, werden vorteilhafterweise die Signale mehrerer Zellen des abgeschirmten Teils des Detektorfeldes zusammengefasst, um hieraus ein gemitteltes Signal zu bilden. Auf diese Weise können die Werte für die Streustrahlung geglättet und Fehlinterpretationen durch zufällige Schwankungen vermieden werden.

Die Streustrahlungskorrektur kann an einem Punkt des direkt bestrahlten Teils des Detektorfeldes dadurch vorgenommen werden, dass von dem Messwert dieses Punktes diejenige Strahlungsintensität subtrahiert wird, welche in der Zelle des abgeschirmten Teils des Detektorfeldes gemessen wurde, die dem betrachteten Punkt am nächsten liegt. Alternativ können auch zwei oder mehr Zellen des abgeschirmten Teils des Detektorfeldes zusammengefasst und der Mittelwert ihrer Messsignale gebildet werden, wobei die hierzu herangezogenen Zellen diejenigen sind, die in größter räumlicher Nähe zum betrachteten Punkt des direkt bestrahlten Teils des Detektorfeldes liegen. Schließlich ist es auch möglich, die im gesamten abgeschirmten Teil des Detektorfeldes gemessenen Streustrahlung durch eine Interpolation bzw. Extrapolation bei der Streustrahlungskorrektur an einem Punkt zu berücksichtigen. Dabei können z.B. die Parameter für ein das gesamte Detektorfeld abdeckendes mathematisches Modell der Streustrahlungsverteilung mit Hilfe der gemessenen Werte im abgeschirmten Teil angepasst werden.

Da die Verteilung der Streustrahlung im abgeschirmten Teil des Detektorfeldes möglicherweise etwas anders ist als im nicht abgeschirmten Teil, wird bei der Streustrahlungskorrektur vorteilhafterweise ein Kalibrierungsfaktor verwendet, welcher diesen Unterschied ausgleicht. Der Kalibrierungsfaktor lässt sich in verhältnismäßig einfacher Weise experimentell ermitteln oder theoretisch abschätzen. Der Kalibrierungsfaktor kann auch die lokal unterschiedlich großen Raumwinkel berücksichtigen, aus denen Streustrahlung eintreten kann.

Vorzugsweise wird die Messanordnung bei der Herstellung eines Computertomogramms auf einer Spiralbahn um die Achse des Objektes verschoben. Durch ein derartiges Verfahren wird ein kontinuierlicheres Erzeugen des Computertomogramms mit einer reduzierten Strahlenbelastung für den Patienten möglich. Die besondere Aufnahmeform bedingt dabei, dass nur ein nicht rechtwinklig begrenzter Teil des Detektorfeldes für die Auswertung und die Erzeugung des Bildes erforderlich ist. Der nicht benötigte Teil des Detektorfeldes wird daher durch geeignet geformte Kollimatoren abgeschirmt, um die Strahlenbelastung für den Patienten zu minimieren. In einer derartigen Anordnung stehen bislang ungenutzte abgeschirmte Teile des Detektorfeldes zur Verfügung, welche erfindungsgemäß zur Streustrahlungskorrektur eingesetzt werden.

Die Erfindung betrifft weiterhin einen Röntgen-Computertomographen enthaltend
- eine Messanordnung bestehend aus einer Röntgenstrahlungsquelle und einem zweidimensional mehrzelligen Detektorfeld, wobei die Messanordnung vorzugsweise in eine Richtung verschiebbar ist,
- Abschirmungsmittel, die so angeordnet sind, dass sie einen nicht rechtwinklig begrenzten Teil des Detektorfeldes abschirmen, und
- eine Korrektureinheit zur Streustrahlungskorrektur der im Detektorfeld gemessenen Strahlungsintensität.

Der Röntgen-Computertomograph ist dadurch gekennzeichnet, dass die Korrektureinheit mit dem abgeschirmten Teil des Detektorfeldes gekoppelt und so eingerichtet ist, dass sie die in diesem Teil gemessene Strahlungsintensität bei der Streustrahlungskorrektur verwendet. Selbstverständlich kann die Korrektureinheit auch mit dem gesamten Detektorfeld gekoppelt sein, wobei sie jedoch für die Streustrahlungskorrektur erfindungsgemäß Gebrauch von den im abgeschirmten Teil gemessenen Strahlungsintensitäten macht.
Mit dem genannten Röntgen-Computertomographen lassen sich die Vorteile des oben geschilderten Verfahrens erzielen. Hierzu gehört insbesondere eine Verbesserung der Bildqualität durch eine Streustrahlungskorrektur, die unter Ausnutzung abgeschirmter Teile des Detektorfeldes die tatsächliche Größe der Streustrahlung lokal aufgelöst erfasst. Die mit der Vorrichtung erzielbare Streustrahlungskorrektur ist deutlich besser als die üblicherweise angewendete Subtraktion eines abgeschätzten konstanten Wertes für den Streustrahlungshintergrund.

Vorzugsweise ist die Korrektureinheit des Röntgen-Computertomographen so eingerichtet, dass sie die verschiedenen Ausgestaltungen des oben erläuterten Verfahrens ausführen kann. Insbesondere kann sie so eingerichtet sein, dass sie nur vollständig abgeschirmte Zellen des Detektorfeldes bei der Streustrahlungskorrektur berücksichtigt, dass sie die Messwerte von mehreren benachbarten Zellen des Detektorfeldes mittelt und diese Mittelwerte bei der Streustrahlungskorrektur berücksichtigt, und/oder dass sie eine Streustrahlungskorrektur an einem Punkt des direkt bestrahlten Teils des Detektorfeldes durch Subtraktion einer interpolierten/extrapolierten Streustrahlungsintensität vornimmt (z.B. derjenigen, die in der nächstgelegenen Zelle oder im Mittel in mehreren nächstgelegenen Zellen des abgeschirmten Teils des Detektorfeldes vorliegt).

Gemäß einer anderen Weiterbildung des Röntgen-Computertomographen ist die Korrektureinheit mit den Abschirmungsmitteln so gekoppelt, dass die Korrektureinheit von den Abschirmungsmitteln Informationssignale betreffend die Form und Größe des abgeschirmten Bereiches des Detektorfeldes erhält. Eine solche Kopplung ist insbesondere dann sinnvoll, wenn sich die Abschirmung des Detektorfeldes je nach angewendetem Scanningverfahren oder in der Abhängigkeit von sonstigen Randparametern ändert. In diesem Falle kann die Korrektureinheit die aktuelle und tatsächlich vorliegende Form und Größe des abgeschirmten Teils des Detektorfeldes automatisch von den Abschirmungsmitteln mitgeteilt bekommen, so dass sie die richtigen Zellen des Detektorfeldes bei der Streustrahlungskorrektur berücksichtigen kann.

Die Erfindung betrifft weiterhin ein Verfahren zur Erzeugung eines Röntgen-Computertomogramms eines Objektes, bei dem das Objekt in einer Messanordnung bestehend aus einer Röntgenstrahlungsquelle und einem Detektorfeld durchstrahlt wird und die im Detektorfeld gemessene Strahlungsintensität einer Streustrahlungskorrektur unterzogen wird. Das Verfahren ist dadurch gekennzeichnet, dass bei der Streustrahlungskorrektur die Ergebnisse einer Computersimulation der Streuprozesse berücksichtigt werden.

Bei dem erfindungsgemäßen Verfahren kann eine erheblich bessere Streustrahlungskorrektur erreicht werden, als sie bei der üblicherweise angewendeten Subtraktion eines konstanten Streustrahlungshintergrundes möglich ist. Anders als bei dem aus der US 5 615 279 bekannten Verfahren sind dabei nicht aufwendige Experimente zur Ermittlung der Streustrahlung erforderlich, sondern die sich einstellende Größe der Streustrahlung kann vielmehr mit großer Genauigkeit und unter Berücksichtigung zahlreicher Einflussparameter in einer Computersimulation bestimmt werden. Dies hat weiterhin den Vorteil, dass bei einer eventuellen Änderung von Parametern wie etwa einem anderen geometrischen Aufbau der Messanordnung die Computersimulation ohne größeren Aufwand mit anderen Randparametern erneut durchgeführt werden kann, so dass flexibel auf derartige Änderungen reagiert werden kann.

Die Computersimulationen der Streuprozesse werden vorzugsweise mit Hilfe eines Monte-Carlo-Verfahrens durchgeführt. Dabei werden die Bahnen zahlreicher virtueller Photonen in der Simulation berechnet und verfolgt. Das Monte-Carlo-Prinzip beruht auf der Berechnung von Zufallsgrößen, die z.B. Richtungsänderungen der virtuellen Photonen bestimmen oder darüber entscheiden, ob ein Photon absorbiert oder gestreut wird. Von diesen Zufallsgrößen sind die Verteilungsfunktionen und/oder Verteilungsdichten bekannt und werden entsprechend angesetzt. Die Behandlung von Strahlungstransportproblemen mit Monte-Carlo-Verfahren ist grundsätzlich aus der Astrophysik und aus der Nuklearphysik bekannt, so dass bezüglich theoretischer Grundlagendetails hierauf verwiesen werden kann. Nach Ausrechnen einer hinreichend großen Anzahl individueller Photonenbahnen erhält man eine Verteilung der auf den Detektor eintreffenden gestreuten Photonen, welche näherungsweise der tatsächlich eintretenden Verteilung entspricht.

Bei der Computersimulation der Streuprozesse werden insbesondere die Geometrie und die Materialeigenschaften der Messanordnung (Röntgenquelle, Kollimatoren und Detektorfeld), des Patiententisches und gegebenenfalls weiterer an Streuprozessen beteiligter Gegenstände berücksichtigt. Die Geometrie und die Materialeigenschaften dieser Gegenstände sind genau bekannt. Die parametrisierte Erfassung dieser Größen in der Computersimulation ermöglicht es bei einer eventuellen Änderung des Aufbaus, durch eine Wiederholung der Simulation mit den geänderten Daten eine angepasste Streustrahlungskorrektur zu berechnen.

Weiterhin werden in der Computersimulation vorzugsweise die Geometrie und die Materieeigenschaften eines Modells des Patientenkörpers im durchstrahlten Bereich berücksichtigt. Auf diese Weise kann die Abhängigkeit der Streustrahlung von den untersuchten Gewebearten, zum Beispiel Gehirnmitte, Leber, Hüfte etc., erfasst werden. Grundsätzlich kann die Streustrahlung auch in Abhängigkeit von internen Strukturen des Körpers wie zum Beispiel der Knochenverteilung berechnet werden. Das niederfrequente Verhalten der Streustrahlung mittelt derartige Strukturen in der Regel soweit aus, dass sie in der Abbildung nicht mehr erkennbar sind. Die genannten Computersimulationen mit Hilfe eines Modells des Patientenkörpers werden vorzugsweise für verschiedene Größen des Modells durchgeführt, so dass bei der späteren Untersuchung eines Patienten die Daten zugrunde gelegt werden können, die mit einem entsprechenden Modell gewonnen wurden. Dabei können die Ergebnisse der Computersimulation insbesondere auch durch Multiplikation mit mindestens einem geeigneten Skalierungsfaktor an die genaue Größe des Patienten angepasst werden.

Ferner kann die Computersimulation die Wechselwirkung der Streustrahlung mit dem Detektor berücksichtigen. Der Detektor oder Szintillator reagiert in der Regel unterschiedlich empfindlich auf Photonen unterschiedlicher Energien. Der Störeinfluss, den gestreute Photonen auf das Signal einer Detektorzelle ausüben, hängt somit auch von der Energie dieser Photonen ab. Ein detaillierteres Computersimulationsmodell berücksichtigt daher auch, mit welcher Energie die gestreuten Photonen am Detektor eintreffen und welches Signal sie im Detektor hervorrufen.

Weiterhin kann das in der Computersimulation verwendete Modell dadurch verbessert werden, dass auch Rückstreuung von Photonen aus dem (Halb-) Raum berücksichtigt wird, welcher von der Strahlungsquelle aus gesehen hinter dem Detektorfeld liegt. Photonen, welche durch das Detektorfeld hindurch getreten oder hieran vorbei geflogen sind, können durch anschließend in dem rückwärtigen Raum stattfindende Streuprozesse zum Detektorfeld zurück gelenkt werden und dort ein Signal hervorrufen. Eine Verbesserung der Computersimulation ist daher möglich, wenn auch derartige Beiträge von rückgestreuten Photonen berücksichtigt werden.

Die Ergebnisse der Computersimulationen werden vorzugsweise für verschiedene Randparameter, die insbesondere die Geometrie der Messanordnung, das eingesetzte Messverfahren und die Objektgröße betreffen, in einer Lookup-Tabelle gespeichert. Aus einer derartigen Tabelle können die relevanten Daten wie insbesondere die berechnete Größe der Streustrahlung an einem gegebenen Punkt des Detektorfeldes leicht abgerufen werden. Bei diesem Abruf lassen sich die bei der aktuellen Messung vorliegenden Parameter wie zum Beispiel die Patientengröße berücksichtigen, um auf die jeweils zutreffenden Daten zurückzugreifen.

Die Erfindung betrifft weiterhin einen Röntgen-Computertomographen mit einer Messanordnung, die eine Röntgenstrahlungsquelle und ein Detektorfeld enthält, sowie mit einer Korrektureinheit zur Streustrahlungskorrektur der im Detektorfeld gemessenen Strahlungsintensität. Der Röntgen-Computertomograph ist dadurch gekennzeichnet, dass die Korrektureinheit zur Durchführung eines Verfahren der oben erläuterten Art eingerichtet ist. Das heißt, dass diese Korrektureinheit bei der Streustrahlungskorrektur die Ergebnisse einer Computersimulation der Streuprozesse berücksichtigt. Diese Computersimulation kann bei entsprechender Ausstattung der Korrektureinheit mit Rechenleistung von der Korrektureinheit selbst durchgeführt werden. Vorzugsweise wird die Computersimulation jedoch separat (offline) auf dafür geeigneten Computern durchgeführt, und nur die Ergebnisse der Computersimulation werden der Korrektureinheit in Tabellenform oder auf ähnliche Weise bereitgestellt. Die Ergebnisse können dabei die Einflüsse verschiedener Randparameter wie der Geometrie und der Materieeigenschaften der Messanordnung, des Patiententisches, der Umgebung, eines Modells des Patientenkörpers und dergleichen berücksichtigen.

Die Korrektureinheit enthält vorzugsweise einen Speicher zur Ablage einer Lookup-Tabelle, in welcher die Ergebnisse mindestens einer Computersimulation gespeichert sind. Vorzugsweise sind in der Lookup-Tabelle die Ergebnisse mehrerer Computersimulationen gespeichert, wobei sich die einzelnen Simulationen in der Variation eines Parameters wie zum Beispiel der Größe des Patientenmodells unterscheiden. In einer konkreten Situation bei der Erzeugung eines Computertomogramms kann die Korrektureinheit dann gezielt auf die passende Computersimulation in der Lookup-Tabelle zugreifen.

Die Korrektureinheit wird vorzugsweise durch mindestens einen digitalen Signalprozessor (DSP) realisiert. Digitale Signalprozessoren sind für die schnelle Durchführung von Signalkonvertierungen optimiert. Ein System mehrerer DSPs kann dabei derzeit Datenraten von etwa 1 Gbit/s erreichen. Bis zur 100 eindimensionale Streustrahlungsverteilungen können im internen Speicher eines DSP gespeichert werden. Da die Zugriffszeiten auf externen Speicher etwa so kurz wie für internen Speicher sind, kann darüber hinaus eine erhöhte Anzahl von Verteilungen in Lookup-Tabellen im externen Speicher abgelegt werden, was die Echtzeitverarbeitung der gemessenen Profile nicht beeinträchtigt. Ferner ist es möglich, zwei, vier oder mehr DSPs für eine verbesserte Leistungsfähigkeit miteinander zu koppeln.

Im Folgenden wird die Erfindung mit Hilfe der Figuren beispielhaft erläutert. Es zeigen:
- Fig. 1: die Erzeugung eines Computertomogramms in einer schematisierten Querschnittsansicht;
- Fig. 2: ein zweidimensionales Detektorfeld mit verschiedenartigen abgeschirmten Bereichen;
- Fig. 3: Primärstrahlung und Streustrahlung an einem kugelförmigen Testkörper;
- Fig. 4: das aus den Daten von Figur 3 ermittelte Querschnittsbild des Testkörpers und den Einfluss von verschiedenen Verfahren zur Streustrahlungskorrektur hierauf;
- Fig. 5: simulierte Kurven für den Streustrahlungshintergrund an einem elliptischen Objekt für eine Drehung um 0°, 30°, 60° und 90° um das Objekt herum.

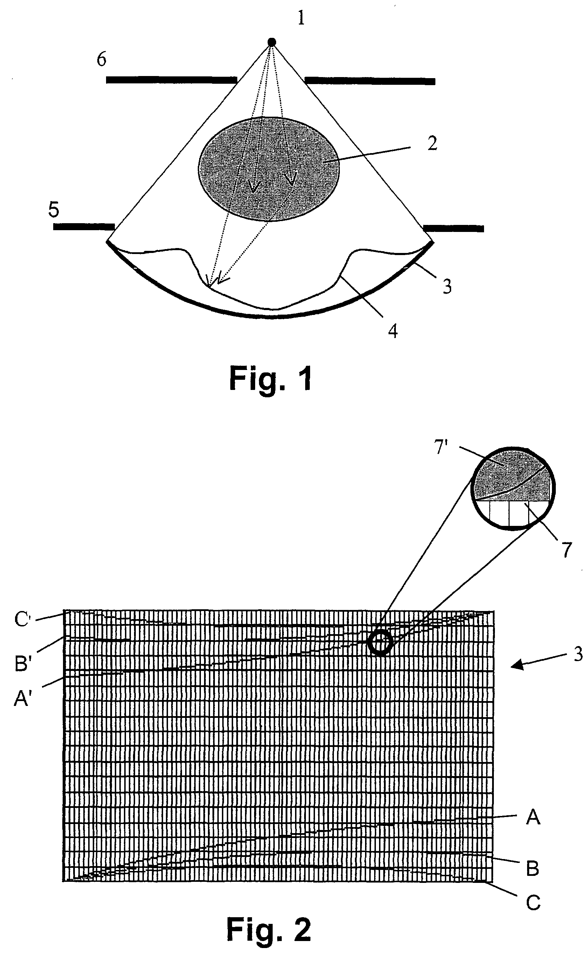

Figur 1 zeigt schematisch die Situation bei der Erzeugung eines Computertomogramms. Hierbei wird Röntgenstrahlung von einer Röntgenstrahlungsquelle 1 in einen Sektor ausgestrahlt, der durch bei der Strahlungsquelle 1 gelegene Kollimatoren 6 begrenzt ist. Die Kollimatoren 6 dienen insbesondere der Strahlbegrenzung in Richtung senkrecht zur Zeichenebene. Die Dicke des bestrahlten Sektors (senkrecht zur Zeichenebene) beträgt typischerweise 0.8 bis 3 mm, wobei zukünftig jedoch tendenziell höhere Schichtdicken von bis zu 2 cm angestrebt werden. Die Röntgenstrahlung durchtritt sodann ein zu untersuchendes Objekt 2, z.B. den Körper einen Patienten. Auf der anderen Seite des Objektes ist ein für Röntgenstrahlung empfindliches Detektorfeld 3 angeordnet, in welchem die Intensität der eintreffenden Röntgenstrahlung ermittelt wird. Das Detektorfeld 3 besteht in der Regel aus nebeneinander liegenden einzelnen Detektorzellen, so dass sich die in Figur 1 erkennbare, mit der Röntgenstrahlungsquelle 1 als Mittelpunkt etwa kreisförmig verlaufende lineare Erstreckung ergibt. Das Detektorfeld 3 kann senkrecht hierzu (das heißt senkrecht zur Zeichenebene) weitere Zeilen mit Detektorzellen enthalten, so dass eine zweidimensionale, mehrzellige Detektormatrix entsteht.

Vor dem Detektorfeld 3 kann ein weiterer Kollimator 5 angeordnet sein, der die Größe des vom ersten Kollimator 6 erzeugten Halbschattens verringern kann. Da dieser hinter dem Objekt befindliche Kollimator 5 jedoch auch Streustrahlung absorbiert, wird im Rahmen der vorliegenden Erfindung vorzugsweise auf seinen Einsatz verzichtet.

Die in dem Detektorfeld 3 eintreffende Röntgenstrahlung besteht zum einen aus direkter Strahlung, die ohne eine Wechselwirkung mit dem Objekt 2 oder der Umgebung von der Röntgenstrahlungsquelle 1 zum Detektor 3 gelangt. Ein Teil der Photonen wird im bestrahlten Objekt absorbiert und fehlt somit bei der direkten Strahlung. Das Ausmaß der Absorption ist abhängig von der Länge des Weges des Strahls durch das Objekt und der Materiezusammensetzung entlang dieses Weges. Diese Absorption enthält die für die Erstellung des Computertomogramms erforderliche Information.

Des weiteren treten bei der Wechselwirkung der Röntgenstrahlung mit der Materie Streuprozesse auf, wobei im für diagnostische Zwecke interessanten Energiebereich von 10 - 140 keV drei verschiedene Interaktionsprozesse vornehmlich relevant sind: Die Photoabsorption (mit nachfolgender K-Fluoreszenzemission), die kohärente und die inkohärente Streuung. Die beiden zuletzt genannten Prozesse vernichten das wechselwirkende Photon nicht, ändern jedoch seine Richtung.

Ein Teil der gestreuten Photonen erreicht ebenfalls das Detektorfeld 3 und trägt dort zu dem in einer Detektorzelle gemessenen Intensitätssignal bei. Da die Detektorzelle nicht differenzieren kann, ob das Photon aus einem Streuprozess stammt oder direkt von der Strahlungsquelle 1 kommt (Primärphoton), verhindern die gestreuten Photonen die exakte Messung der Absorption auf dem direkten Weg zur Strahlungsquelle. Die Streustrahlung beeinträchtigt somit die erzielbare Güte des bildgebenden Verfahrens. Man versucht daher, durch Anti-Streu-Gitter das Eintreffen der Streustrahlung auf dem Detektorfeld 3 zu verhindern. Alternativ wird versucht, den Einfluss der Streustrahlung möglichst genau abzuschätzen, so dass eine Streustrahlungskorrektur mit den Messdaten durchgeführt werden kann.

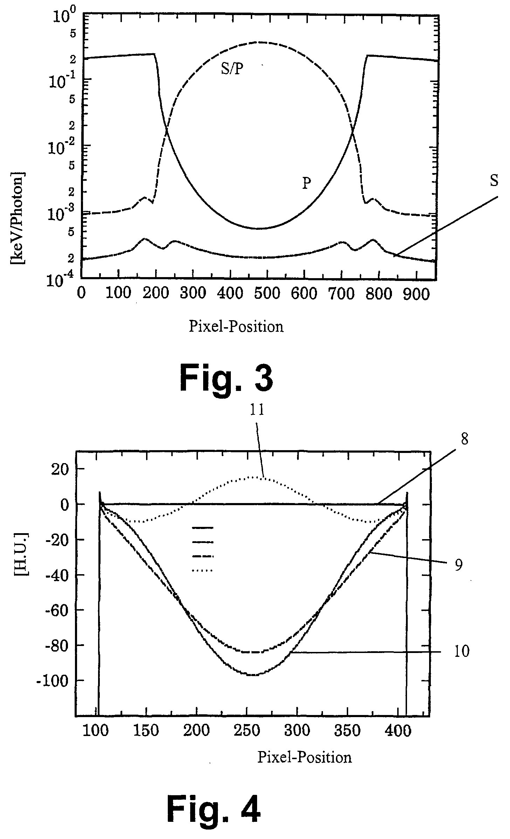

In Figur 1 ist ferner die sich einstellende Streustrahlungsverteilung 4 schematisch angedeutet. Es ist erkennbar, dass die absolute Größe der Streustrahlung an den Rändern des bestrahlten Objektes 2 ansteigt. Das Verhältnis von gestreuter zu primärer Strahlung (SPR) hat jedoch sein Maximum im Zentrum des projizierten Objektes 2, da dort aufgrund der Absorption die Primärstrahlung minimal ist (vgl. unten Figur 3).

In Figur 2 ist ein zweidimensionales Detektorfeld 3 dargestellt, in welchem Detektorzellen 7, 7' matrixartig in zwei Dimensionen nebeneinander angeordnet sind. Ein derartiges Detektorfeld wird für verschiedene Rekonstruktionsalgorithmen benötigt, bei denen das Detektorfeld 3 asymmetrisch bestrahlt wird. Derartige Algorithmen wie zum Beispiel der sogenannte Pi-Linienalgorithmus oder das PHI-Verfahren erfordern eine besondere Form des Detektorfeldes, die nicht rechtwinklig begrenzt ist, sondern in der Regel krummlinig begrenzte Seiten aufweist. Da die genaue Form der benötigten Detektorfläche von den Erfassungsparametern abhängt, wird sie vorzugsweise flexibel durch bei der Strahlungsquelle und/oder dem Detektorfeld angeordnete Kollimatoren 5, 6 definiert. Die Kollimation ist dabei erforderlich, um eine unnötige Bestrahlung des Patienten zu vermeiden und die Strahlungsdosis somit zu minimieren.

In Figur 2 sind die sich für verschiedene Algorithmen ergebenden abgeschirmten bzw. direkt bestrahlten Detektorflächen durch ihre Ränder A, B, C, A', B', C' dargestellt. So wird zum Beispiel die zwischen den gekrümmt verlaufenden Linien A und A' liegende Fläche bei einem Verfahren direkt bestrahlt, bei dem die Messanordnung aus Strahlungsquelle und Detektorfläche spiralförmig um den Patienten herum geführt wird.

Wie aus Figur 2 weiterhin erkennbar ist, werden bei den zugrunde liegenden Algorithmen zahlreiche Zellen 7' des Detektorfeldes keiner direkten Bestrahlung ausgesetzt. Gegebenenfalls tritt im Randbereich des direkt bestrahlten Detektorfeldes eine Schattenzone aufgrund der endlichen Ausdehnung der Strahlungsquelle auf. Diese Schattenzone kann durch Kollimatoren beim Detektor jedoch begrenzt oder minimiert werden. In jedem Falle sind bei den Anordnungen Detektorzellen 7' vorhanden, welche ausschließlich von Streustrahlung getroffen werden können. Das Signal dieser von der direkten Bestrahlung abgeschirmten und nur von Streustrahlung getroffenen Detektorzellen 7' wird erfindungsgemäß dazu verwendet, eine Streustrahlungskorrektur bei denjenigen Zellen 7 auszuführen, die im direkt bestrahlten Bereich des Detektorfeldes 3 liegen. Die direkte Messung der Streustrahlung hat dabei gegenüber der Subtraktion eines konstanten Streustrahlungshintergrundes den Vorteil, dass sie nicht geschätzt, sondern für die jeweilige Situation gemessen wird, und dass sie eine ortsaufgelöste Verteilung entlang der Achse des Detektorfeldes 3 liefert. Wie aus der Teilvergrößerung von Fig. 2 erkennbar ist, kann die gemessene Intensität in einer direkt bestrahlten Detektorzelle 7 durch die Streustrahlungsgröße korrigiert werden, die in der nächstgelegenen abgeschirmten Detektorzelle 7' gemessen wird. Alternativ kann auch ein parametrisiertes globales mathematisches Modell für die Streustrahlungsverteilung an die gemessenen Werte angepasst werden, so dass die Streustrahlungskorrektur dann mit dem Modell bestimmt werden kann. Das Modell kann insbesondere auch aus einer Simulation der Streuung virtueller Photonen bestehen.

Figur 3 zeigt simulierte Werte für die primäre Strahlung P und die gestreute Strahlung S bei der Untersuchung einer Wasserkugel von 30 cm Durchmesser mit einem 60 keV Röntgenstrahl. Auf der horizontalen Achse ist die Pixel-Position des Detektors 3 (Figur 1) und auf der vertikalen Achse die Energieflussdichte (in keV pro Photon) aufgetragen. Die durchgezogene Kurve P stellt die gemessene Intensität der primären (direkten) Photonen dar, welche zum Mittelpunkt des Detektorfeldes hin auf ein Minimum abnimmt. Dieser Verlauf ist dadurch erklärlich, dass die Absorption der primären Photonen aufgrund der vom Rand zum Mittelpunkt der Kugel hin zunehmenden Weglänge stetig wächst.

Die mit kürzeren Strichen gezeichnete Kurve S zeigt die Intensität der gestreuten Photonen. In dermit längeren Strichen ausgeführten Kurve ist das Verhältnis S/P von Primärstrahlung zu gestreuter Strahlung dargestellt. Erkennbar ist hier, dass dieses Verhältnis in der Mitte unter der Kugel ein Maximum annimmt. Wenn daher wie bei üblichen Verfahren ein konstanter Hintergrund an Streustrahlung angenommen und vom Signal der Primärphotonen abgezogen wird, ergibt sich im rekonstruierten Bild ein sogenannter Tasseneffekt. Das heißt, dass die Streustrahlung zu den Rändern des Objektes hin überschätzt wird, was wiederum zu einer Überschätzung der Absorption führt. Die Ränder des Objektes werden somit im Vergleich zum Zentrum des Objektes optisch zu dicht rekonstruiert. Dies führt zu störenden Tassenformen oder Streifen im rekonstruierten Querschnitt.

In Figur 4 ist die Rekonstruktion des Schnittes durch die Kugel dargestellt, deren simulierte Vermessung Figur 3 zeigt. Ohne Streustrahlung müsste sich die durchgezogene Linie 8 ergeben, welche einer konstanten, durch die gesamte Kugel homogenen Dichte entspricht. Die mit kurzen Strichen ausgeführte Linie 10 zeigt eine Verschiebung mit der Annahme eines konstanten Streuhintergrundes mit einem Verhältnis von Streuung zu Primärstrahlung von 37% bei dem kleinsten Primärstrahlungssignal an. Die mit längeren Strichen ausgeführte Kurve 9 zeigt das Ergebnis einer in Computersimulationen berechnete Streustrahlung mit einem maximalen Verhältnis von Streustrahlung zu Primärstrahlung von 37%. Die gepunktete Linie 11 zeigt die resultierende Rekonstruktion, wenn die konstante Streustrahlungsverschiebung von dem simulierten Hintergrund abgezogen wird. Die weiterhin präsenten Abweichungen belegen den Effekt einer zu einfachen Streustrahlungskorrektur.

Die Computersimulation der Streustrahlung erfolgt vorzugsweise unter Verwendung eines Monte-Carlo basierten Simulationsmodells der Photonenwechselwirkungen. Aus den Simulationen kann eine Sammlung berechneter Streuungsverteilungen für eine Vielzahl von Strahlungsquellen und Strahlparametern für verschiedene Bereiche des menschlichen Körpers berechnet werden, die typischen Tomographie-Szenarien entsprechen, und die bei der Erstellung einer Lookup-Tabelle verwendet werden können. Bei der Berechnung der Streuprozesse können insbesondere die folgenden Effekte berücksichtigt werden:
a) die Wechselwirkung der Röntgenstrahlung mit dem Patienten und dem Patiententisch,
b) die Wechselwirkung mit der mechanischen Umgebung (Kollimatoren, Anti-Streu-Gitter, Detektorabdeckung etc.),
c) die Energiedeposition der Strahlung im Szintillator, und
d) Rückstreuungseffekte von durchgelassenen Photonen.

Unterschiede in den Patientengrößen können durch Skalierungsfaktoren berücksichtigt werden. Mögliche Unterschiede zwischen den Simulationen und realen Messungen können darüber hinaus durch gemessene Normierungsfaktoren aufgefangen werden. Durch den Einsatz einer Computersimulation kann unter Bereitstellung der notwendigen Information über das Scanningszenario eine sehr präzise Korrektur der Streustrahlung vorgenommen werden. Die berechneten Ergebnisse können dabei in einer Lookup-Tabelle für Streustrahlungsverteilungswerte gespeichert werden, welche typischerweise in spezieller Hardware wie zum Beispiel einer DSP-Korrekturplatine oder einem schnellen PC für Echtzeitkorrektur realisiert werden.

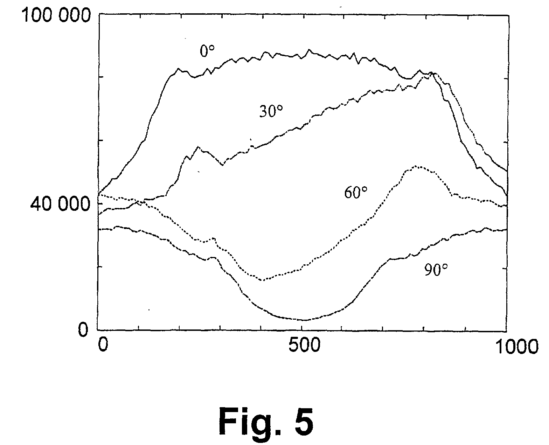

Figur 5 zeigt simulierte Kurven des Streustrahlungshintergrundes für ein ellipsoides Objekt, welches zwei Knocheneinfügungen enthält (relative Einheiten der Strahlungsintensität auf der y-Achse; Detektor-Pixelposition auf der x-Achse). Abgesehen von statistischen Fluktuationen zeigen die Kurven das glatte (niederfrequente) Verhalten des Streuungshintergrundes. Die einzelnen Kurven entsprechen einer Rotation von 0°, 30°, 60° und 90° um das Objekt, wobei für 0° die Längsachse des Objektes auf den Detektor projiziert wird.

## Patentansprüche

1. Verfahren zur Erzeugung eines Röntgen-Computertomogramms eines Objektes (2), wobei das Objekt in einer Messanordnung bestehend aus einer Röntgenstrahlungsquelle (1) und einem Detektorfeld (3) durchstrahlt wird und die im Detektorfeld gemessene Strahlungsintensität einer Streustrahlungskorrektur unterzogen wird, und wobei das Detektorfeld (3) zweidimensional mehrzellig ist und ein nicht rechtwinklig begrenzter Teil des Detektorfeldes von der direkten Bestrahlung durch die Röntgenstrahlungsquelle abgeschirmt ist,
**dadurch gekennzeichnet,**
**dass** die Strahlungsintensität im abgeschirmten Teil des Detektorfeldes gemessen und für die Streustrahlungskorrektur der Messwerte im direkt bestrahlten Teil des Detektorfeldes verwendet wird.

2. Verfahren nach Anspruch 1,
**dadurch gekennzeichnet,**
**dass** der abgeschirmte Teil des Detektorfeldes zumindest teilweise krummlinig (A, A'; B, B'; C, C') begrenzt ist.

3. Verfahren nach Anspruch 1 oder 2,
**dadurch gekennzeichnet,**
**dass** nur solche Zellen (7') des abgeschirmten Teils des Detektorfeldes bei der Streustrahlungskorrektur berücksichtigt werden, die vollständig abgeschirmt sind.

4. Verfahren nach mindestens einem der Ansprüche 1 bis 3,
**dadurch gekennzeichnet,**
**dass** die über mindestens zwei Zellen (7') des abgeschirmten Teils des Detektorfeldes (3) gemittelte Strahlungsintensität für die Streustrahlungskorrektur der Messwerte im direkt bestrahlten Teil des Detektorfeldes verwendet wird.

5. Verfahren nach mindestens einem der Ansprüche 1 bis 4,
**dadurch gekennzeichnet,**
**dass** von einem Punkt (7) des direkt bestrahlten Teils des Detektorfeldes diejenige Strahlungsintensität subtrahiert wird, die sich in einer Interpolation oder Extrapolation aus allen oder aus einem Teil aller im abgeschirmten Teil des Detektorfeldes gemessenen Strahlungsintensitäten ergibt, insbesondere diejenige Strahlungsintensität, die in der am engsten benachbarten Zelle (7') oder die im Mittel in mindestens zwei am engsten benachbarten Zellen des abgeschirmten Teils des Detektorfeldes gemessen wurde.

6. Verfahren nach mindestens einem der Ansprüche 1 bis 5,
**dadurch gekennzeichnet,**
**dass** die Messanordnung auf einer Spiralbahn um die Achse des Objektes (2) verschoben wird.

7. Röntgen-Computertomograph mit einer Messanordnung, die eine Röntgenstrahlungsquelle (1), ein Detektorfeld (3) und Abschirmungsmittel (5, 6) für das Detektorfeld enthält, sowie mit einer Korrektureinheit zur Streustrahlungskorrektur der im Detektorfeld gemessenen Strahlungsintensität,
**dadurch gekennzeichnet,**
**dass** die Korrektureinheit mit dem abgeschirmten Teil des Detektorfeldes gekoppelt und so eingerichtet ist, dass sie die in diesem Teil gemessene Strahlungsintensität bei der Streustrahlungskorrektur verwendet.

8. Röntgen-Computertomograph nach Anspruch 7,
**dadurch gekennzeichnet,**
**dass** die Korrektureinheit so eingerichtet ist, dass sie ein Verfahren nach einem der Ansprüche 1 bis 6 ausführen kann.

9. Röntgen-Computertomograph nach Anspruch 7 oder 8,
**dadurch gekennzeichnet,**
**dass** die Korrektureinheit mit den Abschirmungsmitteln (5, 6) so gekoppelt ist, dass sie Informationssignale über die Form und Größe des abgeschirmten Teils des Detektorfeldes erhält.

10. Verfahren, insbesondere nach mindestens einem der Ansprüche 1 bis 6, zur Erzeugung eines Röntgen-Computertomogramms eines Objektes (2), wobei das Objekt in einer Messanordnung bestehend aus einer Röntgenstrahlungsquelle (1) und einem Detektorfeld (3) durchstrahlt wird und die im Detektorfeld gemessene Strahlungsintensität einer Streustrahlungskorrektur unterzogen wird,
**dadurch gekennzeichnet,**
**dass** bei der Streustrahlungskorrektur die Ergebnisse einer Computersimulation der Streuprozesse berücksichtigt werden.

11. Verfahren nach Anspruch 10,
**dadurch gekennzeichnet,**
**dass** die Computersimulation mit Hilfe einer Monte-Carlo-Simulation virtueller Photonenbahnen durchgeführt wird.

12. Verfahren nach Anspruch 10 oder 11,
**dadurch gekennzeichnet,**
**dass** die Computersimulation die Geometrie und die Materieeigenschaften der Messanordnung, des Patiententisches und gegebenenfalls sonstiger an Streuprozessen beteiligter Gegenstände berücksichtigt.

13. Verfahren nach mindestens einem der Ansprüche 10 bis 12,
**dadurch gekennzeichnet,**
**dass** die Computersimulation die Geometrie und die Materialeigenschaften eines Modells des Patientenkörpers im durchstrahlten Bereich berücksichtigt.

14. Verfahren nach Anspruch 13,
**dadurch gekennzeichnet,**
**dass** die Ergebnisse der Computersimulation durch Multiplikation mit mindestens einem Skalierungsfaktor an die Körpergröße des realen Patienten angepasst werden.

15. Verfahren nach mindestens einem der Ansprüche 10 bis 14,
**dadurch gekennzeichnet,**
**dass** die Computersimulation die Wechselwirkung der Streustrahlung mit dem Detektorfeld berücksichtigt.

16. Verfahren nach mindestens einem der Ansprüche 10 bis 15,
**dadurch gekennzeichnet,**
**dass** die Computersimulation Rückstreuung aus dem von der Strahlungsquelle aus gesehen hinter dem Detektorfeld gelegenen Raum berücksichtigt.

17. Verfahren nach mindestens einem der Ansprüche 10 bis 16,
**dadurch gekennzeichnet,**
**dass** die Ergebnisse der Computersimulation für verschiedene Parameter wie die der Geometrie der Messanordnung, des eingesetzten Messverfahrens und der Objektgröße in einer Lookup-Tabelle gespeichert werden.

18. Röntgen-Computertomograph, insbesondere nach mindestens einem der Ansprüche 7 bis 9, mit einer Messanordnung, die eine Röntgenstrahlungsquelle (1) und ein Detektorfeld (3) enthält, sowie mit einer Korrektureinheit zur Streustrahlungskorrektur der im Detektorfeld gemessenen Strahlungsintensität,
**dadurch gekennzeichnet,**
**dass** die Korrektureinheit zur Durchführung eines Verfahrens nach einem der Ansprüche 10 bis 17 eingerichtet ist.

19. Röntgen-Computertomograph nach Anspruch 18,
**dadurch gekennzeichnet,**
**dass** die Korrektureinheit einen Speicher zur Ablage einer Lookup-Tabelle mit Ergebnissen mindestens einer Computersimulation enthält.

20. Röntgen-Computertomograph nach einem der Ansprüche 18 oder 19,
**dadurch gekennzeichnet,**
**dass** die Korrektureinheit mindestens einen digitalen Signalprozessor enthält.
